# EUROPEAN PATENT APPLICATION

(11) **EP 2 277 523 A1**
(43) Date of publication of application: **26.01.2011**
(21) Application number: 10009643.7
(22) Date of filing: 03.08.2005
(51) Int. Cl.: A61K 31/57, A61K 31/635, A61P 11/00

(54) **Use of megestrol acetate for the treatment of obstructive pulmonary diseases**

(62) Divisional of application: 05016875.6
(71) Applicant: Par Pharmaceuticals, Inc., Woodcliff Lake, NJ 07677 (US)
(72) Inventor: Anker, Stefan, 13088 Berlin (DE); Springer, Jochen, 14612 Falkensee (DE)
(74) Representative: Krauss, Jan

(57) **Abstract**

The present invention relates to the treatment of patients suffering from pulmonary diseases, in particular from chronic obstructive pulmonary disease (COPD), bronchial asthma, cystic fibrosis and chronic cough in particular as caused by ACE-Inhibitors using megestrol acetate.

## Description

The present invention relates to the treatment of patients having suffered from pulmonary diseases, such as chronic obstructive pulmonary disease (COPD), bronchial asthma, cystic fibrosis and chronic cough due to any cause or drug therapy, especially acetylcholinesterase (ACE)-inhibitors using megestrol acetate (MA), in particular using megestrol acetate oral suspension (Megace®), more precisely using the advanced formulation of megestrol acetate oral suspension, namely Megace® ES.

### Background of the invention

In the modem society one major health care problem are pulmonary diseases, more precisely bronchial asthma, cystic fibrosis and chronic obstructive pulmonary disease (COPD). It has been suggested that especially COPD will become one of the leading cause of mortality and disability worldwide [Sin DD., Eur Rev Med Pharmacol Sci. 2004 Nov-Dec; 8(6):247-58].

The underlying clinical phenotypes and risk factors of all above mentioned lung diseases vary from each other. For instance, bronchial asthma is often evoked by an acute allergic inflammation of the bronchial mucosa, cystic fibrosis is an autosomal recessive disorder caused by the mutation of the CF transmembrane conductance regulator (CFTR) gene and one of the most important risk factor for COPD seems to be cigarette smoking. In addition, the therapy of chronic heart insufficiency (CHI), hypertensive or other illnesses by the use of acetylcholinesterase (ACE)-inhibitors might result, as a side effect of the drug, in chronic cough.

Despite the differences in the pathophysiology, COPD, bronchial asthma and cystic fibrosis are all inflammatory disorders defined by the presence of pulmonary obstruction, cough, and sputum. While in bronchial asthma airflow limitation is typically periodic and reversible, COPD and cystic fibrosis show a more progressive and only partially reversible lung obstruction [Doherty DE.; Am J Med 2004 Dec 20;117].

Megestrol acetate (MA) is a synthetically produced derivative of the naturally occurring steroid hormone progesterone. Megestrol acetate is a white, crystalline substance having the chemical name 17-α-acetoxy-6-methylpregna-4,6-diene-3,20-dione (C₂₄H₃₂O₄) and a molecular weight of 384.5. Megestrol acetate has the following formula:

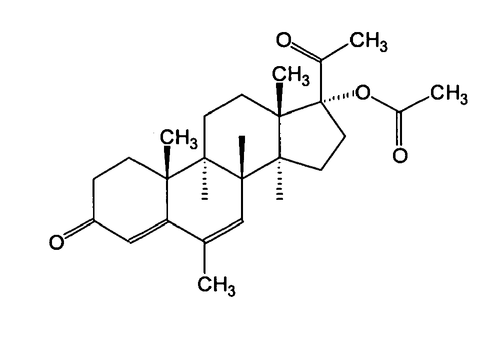

It is known that megestrol acetate is primarily excreted via the kidneys, however, the bioavailability is not completely elucidated. Megestrol acetate is the main agent of the pharmaceutical product Megace® from Bristol-Myers Squibb Company which either has the formulation as an orally active solution or as a tablet.

In clinical medicine megestrol acetate is used as an appetite stimulant that acts by a still unknown mechanism. Initially, megestrol acetate was used for the inhibition of malignant post-menopausal hormone dependent tumours of the breast [Gregory EJ, Cohen SC. Megestrol acetate therapy for advanced breast cancer. J Clin Oncol 1985; 3:155-160. Benghiat A, Cassidy SA. Megestrol acetate in the treatment of advanced post-menopausal breast cancer. Eur J Surg Oncol 1986 12: 43-45, Goss PE. Pre-clinical and clinical review of vorozole, a new third generation aromatoase inhibitor. Report. Breast Cancer Res Tr 1998; 49: S59-S65] and the prostate [Keller J, White JM. A phase III randomised comparative trial of megestrol acetate v. diethylstilbestrol in stage D2 prostatic cancer. Preliminary results. Proc Am Soc Clin Oncol 1986, 5:421. Bonomi P, Pessis D, Bunting N, et al. Megestrol acetate used as primary hormonal therapy in stage D prostatic cancer. Semin Oncol 1985; 12 (Suppl 1): 36-39]. While using it, an increase of weight and an appetite stimulation was discovered as side effects. Initially, a dose of 30 mg/d [Stoll BA. Progestin therapy of breast cancer: comparison of agents. Br Med J 1967; 3: 338-341] was used.

So far, megestrol acetate has been used for several years successfully in cachexia of patients having mamma carcinoma or other malignant tumours. In Germany, this drug is only registered for the therapy in progressed mamma carcinoma. For a specific therapy in patients with mama carcinoma in phase IV for improving the quality of life the dosages 160 mg/d, 800 mg/d and 1600 mg/d were compared in the CALGB-study and 160 mg/d was found as the optimal dosage [Komblinth AB, Hollis DR, Zuckerman E, et al. Effect of megestrol acetate on quality of life in a dose-response trial in woman with advanced breast cancer. J Clin Oncol 1993; 11: 2081-2089].

Recently an advanced formulation of megestrol acetate oral suspension has been introduced, namely Megace® ES. Both oral suspensions contain the same active agent, however, Megace® ES has a different formulation why resorption is significantly improved. Par Pharmaceutical Companies, Inc. manufacturs this advanced formulation, disclosed in the PCT application WO 03/086354 A1. The improvement is caused by a nanoparticulate composition comprising megestrol acetate and preferably at least one surface stabilizer associated with the surface of the drug. These nanoparticulate megestrol particles have an effective average particle size of less than about 2000 nm. This advanced formulation utilises NanoCrystal^{™} Technology delivery system to improve the rate of dissolution and bioavailability of the original megestrol acetate oral suspension. NanoCrystal® Technology is a trademark of Elan Pharma International Ltd. Megace® is a registered trademark of Bristol-Myers Squibb Company licensed to Par Pharmaceutical, Inc. The U.S. Food and Drug Administration (FDA) has approved Megace® ES for the treatment of anorexia, cachexia, or an unexplained, significant weight loss in patients with a diagnosis of acquired immunodeficiency syndrome (AIDS). Recent data have shown that the bioavailability of the original formulation is reduced substantially when taken on an empty stomach. With Megace® ES, this reduction in bioavailability is minimized in the fasted state, resulting in improved bioavailability in patients who have not eaten. Megace® ES 625 mg/5 ml and megestrol acetate oral suspension 800 mg/20 ml are bioequivalent in a fed state.

For megestrol acetate, anti-androgene, anti-estrogene and small glucocorticoid-similar effects are assumed [Alexieva-Figusch J, Van Glise HA. Progestin therapy in advanced breast cancer: Megestrol acetate -An evaluation of 160 treated cases. Cancer 1980; 46: 2369-2372]. The aim of pharmacology therapies in obstructive pulmonary diseases depend on the underlying pathogenesis and pathophysiology and therefore vary in the lung disorders bronchial asthma, cystic fibrosis and COPD.

Current therapies in bronchial asthma include, depending on the heaviness of the disorder, either inhaled or peroral applied bronchodilators, such as short-or long-acting beta 2 agonist, mostly in conjunction with corticosteroids. In some cases, anti-leukotriene agents, sustained released methylxanthines, and mast cell stabilizer are indicated.

In the case of cystic fibrosis the use of mucolytics, bronchodilators, and antibiotics are indicated to suppress retention and infection of the mucus plaque at the airway surface.

In COPD, similar to the therapy of bronchial asthma, bronchodilators are used for symptomatic relief. However, unlike bronchial asthma bronchoconstriction in COPD is acetylcholine-mediated, thus anticholinergics are recommend. In the case of contraindication beta 2 agonist are used. In addition, mucolytics, antibiotics, sustained released methylxanthines, and corticosteroids might be indicated. Sometimes vaccinations and immunizations against influenza and pneumococcus are recommended [Sin DD., Eur Rev Med Pharmacol Sci. 2004 Nov-Dec; 8 (6): 247-58].

In addition to the pharmacology treatment, smoking cessation is in every case indicated. For patients being hypoxemic at rest oxygen therapy might be useful.

Improved treatments of pulmonary obstructive diseases (acute or chronic) are always sought for. Thus, object of the present invention is to provide an improved treatment of bronchial asthma, cystic fibrosis and COPD in order to reduce severity, disability and mortality of the diseases. Another object of the invention is the pulmonary protection of patients suffering from side effects of ACE-inhibitors during the therapy of chronic heart insufficiency, hypertension or other illnesses.

The object of the present invention is, in one particular aspect thereof, solved by the use of megestrol acetate or a pharmaceutically acceptable salt thereof, optionally with appropriate adjuvants and additives for the therapy of improvement of pulmonary diseases. In addition, the object of the present invention is solved by the use of megestrol acetate or a pharmaceutically acceptable salt thereof, optionally with appropriate adjuvants and additives for the production of a medicament for the therapy of pulmonary obstructive diseases such as bronchial asthma, cystic fibrosis and COPD. Furthermore, the object of the present invention is solved by the use of megestrol acetate oral solution optionally with appropriate adjuvants and additives for the therapy of the side-effect of ACE-inhibitors used for the treatment of human illness like CHI, hypertension or others.

In the literature there is no claim or evidence that megestrol acetate is a useful drug in the treatment of lung protection and thus in the treatment of pulmonary diseases.

Based on an animal model, the pulmonary influence of the appetite stimulant megestrol acetate in heart failure after a myocardial infarction was analysed. For this, the following factors were particularly taken into account:
1. Histopathology
2. Lung wet weight

Surprisingly, these experiments revealed, that the use of megestrol acetate oral suspension results in a reduction of the inflammation in the great respiratory tract and the number of mucus globet cells. Thus, megestrol acetate exhibits an impact protection on the lung. This effect was particularly (and thus preferably) present when the advanced formulation of megestrol acetate oral suspension (Megace® ES) was used. In order to obtain valid weight measurements, a diuretic was furthermore administered to all animals of the megestrol acetate study. The use of diuretics is a routine measure in patients with heart insufficiency.

Megestrol acetate useable according to the present invention can be provided in any number of forms suitable for administration. Suitable pharmaceutically acceptable forms comprise salts or pre or pro-forms of megestrol acetate.

Examples of pharmaceutically acceptable salts comprise without limitation non toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-p-sulfate derived from p-toluene-sulfonic acid and others. Such salts can be produced by methods known to someone of skill in the art and described in the prior art.

Other salts like oxalate derived from oxalic acid, which is not considered as pharmaceutically acceptable salt can be appropriate as intermediates for the production of megestrol acetate or a pharmaceutically acceptable salt thereof.

Megestrol acetate or a pharmaceutically acceptable salt thereof can be bound to microcarriers or nanoparticles in parenterals like, for example, to finely dispersed particles based on poly(meth)acrylates, polylactates, polyglycolates, polyamino acids or polyether urethanes. Parenteral formulations can also be modified as depot preparations, e.g. based on the "multiple unit principle", if megestrol acetate or a pharmaceutically acceptable salt thereof is introduced in finely dispersed, dispersed and suspended form, respectively, or as a suspension of crystals in the medicament or based on the "single unit principle" if megestrol acetate or a pharmaceutically acceptable salt thereof is enclosed in a formulation, e.g. in a tablet or a rod which is subsequently implanted. These implants or depot medicaments in single unit and multiple unit formulations often consist out of so called biodegradable polymers like e.g. polyesters of lactic and glycolic acid, polyether urethanes, polyamino acids, poly(meth)acrylates or polysaccharides.

Adjuvants and carriers added during the production of the medicaments usable according to the present invention formulated as parenterals are preferably aqua sterilisata (sterilised water), pH value influencing substances like, e.g. organic or inorganic acids or bases as well as salts thereof, buffering substances for adjusting pH values, substances for isotonisation like e.g. sodium chloride, sodium hydrogen carbonate, glucose and fructose, tensides and surfactants, respectively, and emulsifiers like, e.g. partial esters of fatty acids of polyoxyethylene sorbitans (for example, Tween^{®}) or, e.g. fatty acid esters of polyoxyethylenes (for example, Cremophor^{®}), fatty oils like, e.g. peanut oil, soybean oil or castor oil, synthetic esters of fatty acids like, e.g. ethyl oleate, isopropyl myristate and neutral oil (for example, Miglyol^{®}) as well as polymeric adjuvants like, e.g. gelatine, dextran, polyvinylpyrrolidone, additives which increase the solubility of organic solvents like, e.g. propylene glycol, ethanol, N,N-dimethylacetamide, propylene glycol or complex forming substances like, e.g. citrate and urea, preservatives like, e.g. benzoic acid hydroxypropyl ester and methyl ester, benzyl alcohol, antioxidants like e.g. sodium sulfite and stabilisers like e.g. EDTA.

When formulating the medicaments usable according to the present invention as suspensions in a preferred embodiment thickening agents to prevent the setting of megestrol acetate or a pharmaceutically acceptable salt thereof, tensides and polyelectrolytes to assure the resuspendability of sediments and/or complex forming agents like, for example, EDTA are added. It is also possible to achieve complexes of the active ingredient with various polymers. Examples of such polymers are polyethylene glycol, polystyrol, carboxymethyl cellulose, Pluronics^{®} or polyethylene glycol sorbit fatty acid ester. Megestrol acetate or a pharmaceutically acceptable salt thereof can also be incorporated in liquid formulations in the form of inclusion compounds e.g. with cyclodextrins. In particular embodiments dispersing agents can be added as further adjuvants. For the production of lyophilisates scaffolding agents like mannite, dextran, sucrose, human albumin, lactose, PVP or varieties of gelatine can be used.

In as far as megestrol acetate is not included in a liquid drug formulation in its basic form it can be employed within the parenterals in the form of its acid addition salt solvates.

A further important systemic application formulation is peroral administration in the form of tablets, hard or soft gelatine capsules, coated tablets, powders, pellets, microcapsules, compressed oblongs, granulates, cachets, lozenges, chewing gum or sachets. These solid perorally administered formulations can also be formulated as retard and depot systems, respectively. Comprised therein are medicaments with a content of one or more micronised active agents, diffusion and erosion forms based on matrix, e.g. by using fats, waxy or polymeric substances or so called reservoir systems. If the medicament is formulated to release megestrol acetate over a prolonged period of time retarding agents and agents for the controlled release, respectively, can be added like film or matrix forming substances, for example, ethylcellulose, hydroxypropyl methyl cellulose, poly(meth)acrylate derivatives, (e.g. Eurdragit^{®}), hydroxypropyl-methylcellulose phthalate both in organic solutions and in the form of aqueous dispersions. In this context bioadhesive preparations should also be mentioned wherein an extended dwelling time in the body is caused by the intimate contact with the mucous membranes of the body. An example of a bioadhesive polymer is, e.g. the group of Carbomere^{®}.

For the purpose of a controlled release of megestrol acetate or a pharmaceutically acceptable salt thereof within the different segments of the gastro-intestinal tract it is possible to employ a mixture of pellets which release at different locations. The medicament formulation can be coated, for example, with mixtures of films, substances, compounds or compositions soluble in gastric juice and resistant to gastric juice, respectively. The same purpose of affecting the release in different sections of the gastro-intestinal tract can also be reached with appropriately produced coated tablets with a core, wherein the coating releases the active ingredient in gastric juice rapidly and the core releases the active ingredient in the environment of the small intestine. The aim of a controlled release in different sections of the gastro-intestinal tract can also be achieved by multiple coated tablets. Mixtures of pellets with differentially releasable active agent can be filled into, for example, hard gelatine capsules.

A further adjuvant employed in the production of compressed formulations like e.g. tablets, hard and soft gelatine capsules as well as coated tablets and granules are, for example, counter glue agents, lubricating agents and separating agents, dispersion agents like e.g. flame dispersion silicon dioxide, disintegrants like, e.g. various types of starch, PVP, cellulose, ester as granulating or retarding agent like, e.g. waxy and/or polymeric substances based on Eudragit^{®}, cellulose or Cremophor^{®}.

Furthermore medicaments formulated for peroral administration can comprise antioxidants, sweetening agents like, e.g. saccharose, xylite or mannite, taste correcting agents, flavorants, preservatives, colouring agents, buffering agents, direct compression excipients, microcrystalline cellulose, starch, hydrolysed starch (e.g. Celutab^{®}), lactose, polyethylene glycol, polyvinylpyrrolidone, dicalcium phosphate, lubricants, fillers like, e.g. lactose or starch, binders in the form of lactose, types of starch like e.g. wheat or corn and rice starch, respectively, derivatives of cellulose like, e.g. methyl cellulose, hydroxypropyl cellulose or silica, talcum, stearate like, e.g. magnesium stearate, calcium stearate, talkum, siliconised talkum, stearic acid, cetyl alcohol or hydrogenated fats etc. A variety of substances are known to someone of skill in the art which can be added to medicaments for the formulation for peroral administration.

In a further embodiment megestrol acetate or a therapeutically acceptable salt thereof can also be formulated as an oral therapeutic system, in particular based on osmotic principles like, e.g. GIT (gastro-intestinal therapeutic system) or OROS (oral osmotic system).

Effervescent tablets or tabs are also among compressed formulations, which can be perorally administered and which are both rapidly dissolvable or suspendable in water and are rapidly drinkable instant drug formulations.

Perorally administrated formulations also include solutions e.g. drops, juices and suspension which can be produced according to methods known in the art and which can comprise - beside the already mentioned adjuvants and additives for the increase of the stability - preservatives and if desired flavouring agents for easier ingestion and colouring agents for better distinction as well as antioxidants and/or vitamins and sweetening agents like sugars or artificial sweeteners. This also applies to dried juices which are prepared with water prior to use. In a preferred embodiment of a formulation of the medicaments of the present invention an ingestible liquid formulation can also comprise an ion exchange resin.

In a preferred embodiment of the present invention, the megestrol acetate is selected from common megestrol acetate formulations (brand name is Megace®). So far, megestrol acetate has been commercially produced by several companies as generic medicament. For the present study, two formulations of megestrol acetate were used: megestrol acetate oral suspension, below designated as MA-I, and the advanced oral suspension formulation Megace® ES, below designated as MA-II, whereas both preparations were obtained from the company PAR Pharmaceuticals.

Yet another preferred embodiment of the present invention is **characterised in that** the medicament is applied orally. Preferably, megestrol acetate or a pharmaceutical acceptable salt thereof is applied in a dosage of between 30 mg/d and 2000 mg/d, preferably between 100 mg/d and 1600 mg/d, most preferred 300 to 800 mg/d. The active ingredient can be administered in one or several doses per day; alternatively the active ingredient can be administered in larger time intervals. Also preferably, megestrol acetate or a pharmaceutical acceptable salt thereof is applied in a dosage of between 4 and 15 mg/kg/d. The active ingredient can be administered in one or several doses per day; alternatively the active ingredient can be administered in larger time intervals.

In another important embodiment of the present invention, megestrol acetate or a pharmaceutical acceptable salt thereof is applied in combination with drugs typically used in obstructive pulmonary diseases, such as suitable agents.

The invention also relates to a composition comprising megestrol acetate or a pharmaceutical acceptably salt thereof in combination with an ACE-inhibitor.

The compositions according to the present invention comprising one or more suitable bronchdilator, an ACE-inhibitor, a beta 2 agonist, an anticholinergic, glucocorticoid, methylxanthine, mast cell stabilizer, antibiotic, mucolytic, and/or an anti-leukotriene can be produced by someone of skill in the art in one of the formulations disclosed above for megestrol acetate and can be mixed with respectively indicated adjuvants and additives. In a further aspect the invention also relates to the spatially and/or temporally separated administration of the respective active ingredients.

It was a main goal of the study underlying the present invention as well as other studies, to reach reduced inflammation of the respiratory tract and reduced number of mucus globet cells.

Since in most patients having chronic heart insufficiency oedema occur, nearly all human patients with heart insufficiency are undergoing a therapy with a diuretic [Cowie MR, Mosterd A, Wood DA, Deckers JW, Poole-Wilson PA, Sutton GC, et al. The epidemiology of heart failure. Eur Heart J 1997;18:208-225]. Prior studies have shown that also in diseased rats, that are used as a model in present invention, oedema occur which would interfere with the measurements of weight and the comparisons. Thus, all infarct animals were given furosemide together with drinking water. It can therefore be expected that the changes in weight are with a high probability related to an increase or decrease of fat, muscle or bone mass and are not caused by oedema.

In the present invention, a rat infarct model for the production of a chronic heart insufficiency was used. Since, the generation of a CHI in human in any case is caused by a myocardial infarct, this model allows for representative results for CHI. It is known that CHI is a multi-organ disease syndrome with many similarities to other chronic illnesses, such as lung diseases. In addition, CHI very often is accompanied with oedemas and it also might result in a secondary pulmonary hypertension, caused by afflux. Therefore this model is suitable to study the influence of a certain drug on the heart and lung function. The myocardial infarction induced in rats nearly always leads to a proven heart failure in these rats. Rats having an surgically induced myocardial infarction have a lower chance of survival compared to sham-surgery rats. Megestrol acetate significantly improved the survival in infarction surgery rats in comparison to placebo. Besides improved heart function, a reduction in the number of mucus goblet cells and a reduced inflammation of the lung was observed, indicating the pulmonary protective impact of megestrol acetate.

The following example is included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references as cited are incorporated herein by reference in their entireties.

### Example

An animal study was carried out, wherein a ligation of the left coronary artery was performed in 140 animals. For control 20 animals were operated without coronary ligature (sham-surgery). Two weeks after surgery, therapy was started. Therefore, the animals were treated with a diuretic (furosemide, in average 10 per kg in drinking water). After six weeks double-blind randomised therapy with either placebo, old or new megestrol acetate (from day 42 on using gavage, 100 mg per kg,) or ACE-inhibitor (Ramipril®, 1mg per kg per day) was started. The infarct groups consisted of 38 animal each for Ramipril® and placebo, 37 for MA-I and 40 for MA-II, as well as 5 animals per sham group. During the therapy animals were weighed twice a week. On day 224 after surgery animals were sacrificed and the lung was analyzed regarding weight and histopathology.

This study showed surprising results regarding lung function. Megestrol acetate seems to have an pulmonary impact, resulting in lung protection.

### Animals

Male Wistar-rats (Dimed GmbH, Schönwalde, Germany) were held in an environment controlled animal facility having a constant temperature of 23°C in a 12 hour day-night-cycle.

During the whole experiment, all rats were grouped in 1-3 animals per cage. Animals in one cage belong to the same group. The animals had free access to regular food and drinking water or drinking water with diuretic respectively.

### Production of a heart insufficiency via infarction surgery

The animals underwent surgical treatment at a weight of about 210-220 g. In order to produce a heart insufficiency the inventors performed the surgical treatment in accordance with an infarction model that was already used in several prior studies. The heart infarction is produced according to a slightly modified method described by Selye et al. [Selye H, Bajusz E, Grassos S, Mendel P. Simple techniques for the surgical occlusion of coronary vessels in the rat. Angiology 1960; 11: 398-407] by ligation of the LAD.

Each rat was anaesthetised by i.p. chloralhydrate (Merck, Darmstadt, Germany) and was subsequently incubated with a PE-catheter (1.2 mm diameter) and respirated using a Rodent-respirator V5K6 (NarcoBio Systems, Houston, Texas, USA). During the whole operation an ECG was performed as control. The thorax was opened, the heart was prepared and a ligature using Ethibond-suture material 7/0 (Ethicon GmbH, Norderstedt, Germany) was made in position of the left coronary artery (LAD). Since the coronary arteries during this process can not be viewed by the naked eye, the orientation occurred anatomically based on the position of the heart and the visible coronary veins. The success of the infarction could be visually verified based on the occurring paleness, cyanosis and mobility distortion. Subsequently, the thorax was closed. The respiration was ended, once a stable ECG was reached. 20 rats were used as control animals and were surgically treated in the same manner as the infarction animals. No coronary ligature was introduced in these animals (sham-surgery).

After the surgery, the animals were controlled and the weight was analysed twice a week during the whole study. From day 14 on, the animals received furosemide, accompanied from day 42 on with either placebo, ACE-inhibitor, MA-I or MA-II. On day 40 post-surgery the animals were randomised and segregated into the respective groups. During therapy animals were weighted twice a week and on day 224 post-surgery animals were sacrificed to analyze organs.

### Randomisation and blinding

42 days post-surgery, all infarction and control animals were block-randomised and segregated in one of four infarction groups or one of four control groups. Therefore animals received treatment in a blinded fashion, the medication was colour-coded.

### Weight determinations

During the progression of both studies, the animals were weight-determined twice a week, starting from the date of surgery (laboratory balance model CS2000. Ohaus Corporation, Pine Brook, NJ, USA). The results were calculated corresponding to the post-surgical day of the respective animal.

**Table 1: Weight of animals in g at the end of the study**

| | placebo | ramipril | MA-I | MA-II | placebo sham | ramipril sham | MA-I sham | MA-II sham |
|---|---|---|---|---|---|---|---|---|
| number | 11 | 24 | 7 | 25 | 4 | 5 | 4 | 5 |
| mean | 599,3 | 575,0 | 576,3 | 506,6 | 561,5 | 567,2 | 539,0 | 475,6 |
| SD | 36,97 | 39,13 | 39,92 | 53,21 | 44,46 | 34,66 | 76,46 | 78,12 |
| SEM | 11,15 | 7,988 | 15,09 | 10,64 | 22,23 | 15,50 | 38,23 | 34,94 |

**Table 2: Weight of animals normalised to tibia length in g/cm tibia at the end of the study**

| | placebo | Ramipril® | MA-I | MA-II | placebo sham | Ramipril® sham | MA-I sham | MA-II sham |
|---|---|---|---|---|---|---|---|---|
| number | 11 | 24 | 7 | 25 | 4 | 5 | 4 | 5 |
| mean | 139,1 | 133,2 | 139,1 | 126,7 | 129,0 | 135,0 | 133,1 | 117,1 |
| SD | 8,128 | 9,045 | 14,21 | 12,12 | 6,559 | 6,080 | 19,35 | 18,01 |
| SEM | 2,451 | 1,846 | 5,370 | 2,425 | 3,280 | 2,719 | 9,673 | 8,054 |

### Diuretic treatment

All groups of the infarction animals received furosemide (Lasix® 250 mg ampoule, Aventis Pharma GmbH, Frankfurt am Main, Germany) starting from day 14 for the whole study in a concentration of ∼ 10 mg/100 ml in drinking water. This corresponds to an initial dosage of 10 mg/kg/d at a weight of 300 g and an amount of drinking water of 35 ml/d.

All sham-animals received drinking water without supplementation.

### Medication

MA-I, MA-II, ACE-inhibitor (Ramipril®) or control substances were administered to the animals via gavage. Depending on the time of surgery, the animals were gavaged starting from week six after the infarction surgery once a day, whereby the amount of gavage was adjusted to the weight (100 mg MA-I/kg body weight per day, 1 ml solution contains 40 mg MA-I or MA-II or placebo, respectively).

**Table 1: Gavage protocol. 1 ml of the solution contained 40 mg of MA-I or MA-II or placebo, respectively.**

| **Animal weight (g)** | **Gavage (amount)** | **Animal weight (g)** | **Gavage (amount)** |
|---|---|---|---|
| 180-219 | 0.5 ml | 500-539 | 1.3 ml |
| 220-259 | 0.6 ml | 540-579 | 1.4 ml |
| 260-299 | 0.7 ml | 580-619 | 1.5 ml |
| 300-339 | 0.8 ml | 620-659 | 1.6 ml |
| 340-379 | 0.9 ml | 660-699 | 1.7 ml |
| 380-419 | 1.0 ml | 700-739 | 1.8 ml |
| 420-459 | 1.1 ml | 740-779 | 1.9 ml |
| 460-499 | 1.2 ml | 780-819 | 2.0 ml |

### Preparation of organs

In the 32st week post-surgery, the surviving animals were anaesthetised using chloralhydrate i.p. 4 mg/kg body weight (Sigma-Aldrich Chemie GmbH, Deisenhofen, Germany). Following the preparation of the subscapular brown fatty tissue, lapratomisation was performed and blood was drawn from the aorta abdominalis into EDTA-flushed 10 ml syringes (Roth, Karlsruhe, Germany) and added to pre-chilled 12 ml-tubes (Sarstedt, Nümbrecht, Germany). The following parts were removed completely or partially: lung, heart, liver, kidneys, renal glands, inguinal fatty tissue, epididymal fatty tissue, gastrocnemius, and brain.

For histological preparation the parts of the lung, kidney, gastrocnemius, EDL, soleus, adrenal glands were fixed in Zamboni's solution (15% saturated picrinic acid, 2% formaldehyde) [119] and embedded in paraffin. The sections of each organ were stained with AZAN, PAS and Elastica according to standard protocols.

The lung was analysed regarding fibrosis, number of muscus goblet cells in the upper respiratory tracts, the degree of inflammation around the great respiratory tracts and the appearance of an emphysema.

### Statistical Analysis

All results were indicated as mean value ± standard deviation. For statistical analysis the Chiquadrat-test was performed. All calculations were performed using Graph Pad PRISM 4.0 and SPSS 12.0 statistics program.

### Results

### Histopathology of the Lungs

In all animals that could be studied a more or less intensive remodelling of the lung was found. For analysis the fibrosis, the number of mucus goblet cells in the great respiratory tracts, the degree of inflammation around the great respiratory tracts and the appearance of an emphysema was studied.

**Table 1: Numbers of fibrosis, goblet mucus cells, inflammation an emphysema counted in infarction- surgery treated either with MA-I or MA-II, Ramipril®, or placebo.**

| | | | | | |
|---|---|---|---|---|---|
| **Fibrosis** | | | | | |

| | **none** | **little** | **medium** | **strong** | **very strong** |
|---|---|---|---|---|---|
| **Infarct Placebo (n=11)** | 1 | 1 | 4 | 3 | 2 |
| **Infarct Ramipril® (n=23)** | 12 | 5 | 4 | 2 | - |
| **Infarct MA-I (n=7)** | 4 | - | 1 | 2 | - |
| **Infarct MA-II (n=25)** | 12 | 3 | 4 | 6 | - |
| **mucus goblet cells** | | | | | |

| | **none** | **little** | **medium** | **strong** | **very strong** |
|---|---|---|---|---|---|
| **Infarct Placebo (n=11)** | 9 | - | 2 | - | - |
| **Infarct Ramipril® (n=23)** | - | - | 7 | 6 | 10 |
| **Infarct MA-I (n=7)** | 5 | - | 2 | - | - |
| **Infarct MA-II (n=25)** | 20 | - | 5 | - | - |
| **inflammation** | | | | | |

| | **none** | **little** | **medium** | **strong** | **very strong** |
|---|---|---|---|---|---|
| **Infarct Placebo (n=11)** | 2 | - | 4 | 4 | 1 |
| **Infarct Ramipril® (n=23)** | - | 1 | 2 | 2 | 18 |
| **Infarct MA-I (n=7)** | 5 | - | 1 | 1 | - |
| **Infarct MA-II (n=25)** | 12 | 6 | 5 | 2 | - |
| **emphysema** | | | | | |

| | **none** | **little** | **medium** | **strong** | **very strong** |
|---|---|---|---|---|---|
| **Infarct Placebo (n=11)** | 2 | 4 | 4 | 1 | - |
| **Infarct Ramipril® (n=23)** | 8 | 3 | 6 | 4 | 2 |
| **Infarct MA-I (n=7)** | 6 | 1 | - | - | - |
| **Infarct MA-II (n=25)** | 16 | 3 | 4 | 2 | - |

### Statistical analysis of the histopathology of the Lungs

The statistical analysis was performed using the chi-square-test:

| Fibrosis | | |
|---|---|---|
| Overall: X²: 20.18. p = 0.063 | | |
| Placebo vs | Ramipril: | X²: 11.35; p = 0.023 |
| | MA-I: | X²: 6.22; p = 0.183 |
| | MA-II: | X²: 9.26; p = 0.055 |
| Ramipril® vs | MA-I: | X²: 3.16; p = 0.366, combining strong and very strong |
| | MA-II: | X²: 2.42; p = 0.489, combining strong and very strong |
| MA-I vs | MA-II: | X²: 0.98; p = 0.803, combining strong and very strong |

| Mucus goblet cells | | |
|---|---|---|
| Overall: X²: 48.96, p < 0.0001 | | |
| Placebo vs | Ramipril: | X²: 26.89, p < 0.0001 |
| | MA-I: | X²: 0.26, p = 0.605, combining medium, strong and very strong |
| | MA-II: | X²: 0.02, p = 0.899, combining medium, strong and very strong |
| Ramipril® vs | MA-I: | X²: 21.30, p < 0.0001 |
| | MA-II: | X²: 36.31, p < 0.0001 |
| MA-I vs | MA-II: | X²: 0.23, p = 0.628, combining medium, strong and very strong |

| Inflammation | | |
|---|---|---|
| Overall: X²: 60.31, p < 0.0001 | | |
| Placebo vs | Ramipril: | X²: 17.49, p = 0.002 |
| | MA-I: | X²: 5.26, p = 0.260, combining none and little |
| | MA-II: | X²: 11.16, p = 0.025 |
| Ramipril® vs | MA-I: | X²: 22.55, p = 0.0002 |
| | MA-II: | X²: 34.83, p < 0.0001 |
| MA-I vs | MA-II: | X² = 2.57, p = 0.462 |

| Emphysema | | |
|---|---|---|
| Overall: X²: 18.22, p = 0.109 | | |
| Placebo vs | Ramipril: | X²: 4.23, p = 0.375 |
| | MA-I: | X²: 8.32, p = 0.0398, combining strong and very strong |
| | MA-II: | X²: 6.96, p = 0.073, combining strong and very strong |
| Ramipril® vs | MA-I: | X²: 6.64, p = 0.156 |
| | MA-II: | X²: 5.66, p = 0.226 |
| MA-I vs | MA-II: | X² = 2.08, p = 0.556 |

Analyzing the lung using histopathology it was shown that megestrol acetate can be used for pulmonary protection. Since the number of animals treated with MA-I is low compared to the number of animals treated with MA-II, the focus of this analysis regards the MA-II treated animals. However, it should be noted that the results of the MA-I treated animals are in line with the one from the MA-II animals. In 2 of 4 histopathological studies, namely inflammation and mucus goblet cells MA-treatment, in particular MA-II, was significantly better than the placebo group and the Ramipril® group (standard therapy of CHI). In addition, regarding fibrosis and emphysema MA-treatment, in particular MA-II, treatment again seems to have positive effects compared to the standard therapy or placebo group. Although there are no significant differences, the lately trend is very consistent with the other results. All this leads to the assumption that megestrol acetate has a strong impact on pulmonary protection.

### Lung wet weight

In general, the average wet weight of the lungs from the placebo animals was significantly heavier than the average wet weight of the lungs from animals treated either with Ramipril®, old or new megestrol acetate. More precisely, the average wet weight of the lung from placebo animals (n = 30) was significantly heavier than the average wet weight of the lung from MA-II animals (n = 24; 4515 ± 317 mg and 2341 ± 189 mg, respectively; p <0.0001). Thus, MA-II showed a strong impact reducing the development of pulmonary oedema. In line with this the average wet weight of the lung from Ramipril® animals (n = 28; 3103 ± 281 mg) and from MA-II animals (n = 31;3504 ± 249 mg) were also heavier than the average wet weight of the lung from MA-II animals. In summary, the average wet weight of the lung from placebo animals was heavier than the average wet weight of the lung from MA-animals.

A study regarding the amount of liquid in the lung, showed a similar trend. The average weight of the liquid in the lung was from Ramipril®-treated animals (n = 9) and placebo animals (n = 5) was similar (2396 mg ± 212 mg and 2475 mg ± 471 mg, respectively) and compared MA-I (n = 6) and MA-II (n = 10) treated animals heavier (1683 mg ± 113 mg and 1884 mg ± 209 mg, respectively).

## Claims

1. Use of megestrol acetate or a pharmaceutically acceptable salt thereof, optionally with appropriate adjuvants and additives for the production of a medicament for the treatment of pulmonary diseases.

2. Use according to claim 1, **characterised in that** megestrol acetate is selected from common megestrol acetate oral suspension (Megace®) and/or Megace ® ES.

3. Use according to claim 1 or 2, **characterised in that** the pulmonary disease is an obstructive pulmonary disease resulting from bronchial asthma.

4. Use according to claim 3, **characterised in that** the pulmonary disease is an obstructive pulmonary disease resulting from cystic fibrosis.

5. Use according to claim 3, **characterised in that** the pulmonary disease is an obstructive pulmonary disease resulting from chronic obstructive pulmonary disease (COPD).

6. Use according to claim 3, **characterised in that** the pulmonary disease is an obstructive pulmonary disease resulting from acetylcholinesterase (ACE)-inhibitor therapy.

7. Use according to any one of claims 1 to 6, **characterised in that** the medicament is applied orally.

8. Use according to any one of claims 1 to 7, **characterised in that** the megestrol acetate or a pharmaceutical acceptable salt thereof is applied in a dosage of between 30 mg/d and 2000 mg/d, preferably between 100 mg/d and 1600 mg/d, most preferred 300 to 800 mg/d.

9. Use according to any one of claims 1 to 8, **characterised in that** the megestrol acetate or a pharmaceutical acceptable salt thereof is applied in a dosage of between 4 and 15 mg/kg/d.

10. Use according to any one of claims 1 to 7 **characterised in that** the megestrol acetate or a pharmaceutical acceptable salt thereof is applied in combination with drugs used in pulmonary diseases, such as beta 2 agonists, anticholinergics, glucocorticoids, antibiotics, mucolytics, anti-leukotriene agents, mast cell stabilizers and methylxanthines.

11. Use according to any one of claims 1 to 7 **characterised in that** the megestrol acetate or a pharmaceutical acceptable salt thereof is applied in combination with acetylcholinesterase (ACE)-inhibitors for the treatment of chronic cough evoked by ACE-inhibitors.
